(19) Europäisches Patentamt
European Patent Office
Office européen des brevets

(11) Veröffentlichungsnummer: **0 151 400**
**B1**

(12) # EUROPÄISCHE PATENTSCHRIFT

(45) Veröffentlichungstag der Patentschrift: 24.05.89

(51) Int. Cl.⁴: A 61 K 7/13, C 07 C 91/44, C 07 C 93/14, C 07 D 303/36

(21) Anmeldenummer: 85100283.2

(22) Anmeldetag: 12.01.85

(54) Haarfärbemittel mit Pikraminsäurederivaten, neue Pikraminsäurederivate sowie Verfahren zu ihrer Herstellung.

(30) Priorität: 18.01.84 DE 3401560

(43) Veröffentlichungstag der Anmeldung: 14.08.85 Patentblatt 85/33

(45) Bekanntmachung des Hinweises auf die Patenterteilung: 24.05.89 Patentblatt 89/21

(84) Benannte Vertragsstaaten: AT BE CH DE FR GB IT LI LU NL SE

(73) Patentinhaber: Wella Aktiengesellschaft, Berliner Allee 65, D-6100 Darmstadt (DE)

(72) Erfinder: Braun, Hans-Jürgen, Dr., Impasse de La Colline 3, CH-1723 Marly (CH)
Erfinder: Konrad, Eugen, Mecklenburger Strasse 101, D-6100 Darmstadt (DE)
Erfinder: Mager, Herbert, Dr., Beaumont 5, CH-1700 Fribourg (CH)

(56) Entgegenhaltungen:
DE-A-1 444 216
US-A-3 344 031
US-A-3 446 568

CHEMICAL ABSTRACTS, Band 35, Nr. 19, 10. Oktober 1941, Spalten 6726-9 - 6727-6, Columbus, Ohio, US; R.L. METCALF u.a.: "Toxicity and repellent action of some derivatives of picramic acid and of toluenesulfonyl chloride to the greenhouse leaf tyer" & J. ECON. ENTOMOL. 34, 306-9(1941)

(56) Entgegenhaltungen: (Fortsetzung)
JOURNAL OF THE CHEMICAL SOCIETY, PERKIN TRANSACTIONS I, Nr. 2, 1984, Seiten 135-137; SPYROS SPYROUDIS u.a.: "Synthesis and reactivity of 2-oxidodiaryliodonium zwitterions"

Die Akte enthält technische Angaben, die nach dem Eingang der Anmeldung eingereicht wurden und die nicht in dieser Patentschrift enthalten sind.

EP 0 151 400 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Gegenstand der Erfindung sind Mittel zur Färbung von Haaren mit Nitrofarbstoffen, wobei als Nitrofarbstoffe Derivate der Pikraminsäure verwendet werden sowie ein Verfahren zur Herstellung dieser Derivate.

Für die Haarfärbung haben Nitrofarbstoffe eine wesentliche Bedeutung erlangt. Durch Kombination verschiedener Nitrofarbstoffe lassen sich Färbemittel herstellen, die ohne Zusatz von Oxidationsmitteln Haare in natürlichen und modischen Tönen zu färben vermögen. Die Nitrofarbstoffe sind ebenfalls wichtige Bestandteile von Oxidationshaarfärbemitteln, da sie es auf einfache Art ermöglichen, natürliche oder modische Farbnuancen herzustellen.

Neben dem Einsatz in nicht oxidativen Haartönungsmitteln werden orange bis rot färbende Nitrofarbstoffe vor allem in oxidativen Haarfärbemitteln zur Abrundung des Farbergebnisses und zur Erzeugung von modischen Effekten herangezogen.

Insbesondere zur Erzeugung von modischen Roteffekten werden Nitrofarbstoffe benötigt, die das Haar in intensiven Rottönen einzufärben vermögen.

An Nitrofarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Haarfärbungen eine gute Licht-, Säure- und Reibechtheit gefordert. Ihre Verwendung in Oxidationshaarfärbemitteln setzt weiterhin voraus, daß sie in Anwesenheit von Hydrogenperoxid in ammoniakalischer Lösung und gegenüber Antioxidantien stabil sind.

Die Pikraminsäure erfüllt zwar im wesentlichen die vorstehend genannten Voraussetzungen, jedoch lassen sich mit ihr nur Färbungen im Orangebereich erhalten.

In der US-A-3 344 031 sind zwar Isopikraminsäurederivate beschrieben, welche an der Aminogruppe substituiert sind, jedoch färben diese das Haar blauviolett mit geringer Farbintensität. Gegenüber der unsubstituierten Isopikraminsäure, welche das Haar rotviolett färbt, wird der Farbton durch die Substitution an der Aminogruppe zum Blauen hin verschoben.

Das in der DE-A-1 444 216 erwähnte 4-Amino-2-nitro-phenol färbt das Haar lediglich in einem äußerst farbschwachen orangen Farbton.

Weiterhin wird in US-A-3 446 568 ein an der Aminogruppe mit einem 3-Amino-2-hydroxypropylrest substituiertes Pikraminsäurederivat beschrieben, welches das Haar rötlich-braun färben soll. Es hat sich jedoch herausgestellt, daß jene Verbindung, wenn überhaupt, nur sehr schwer hergestellt und rein isoliert werden kann.

Demgegenüber wurde nun gefunden, daß bestimmte, an der Aminogruppe substituierte Derivate der Pikraminsäure die Haare in einem intensiven Rotton färben.

Gegenstand der vorliegenden Patentanmeldung sind daher Mittel zur Färbung von Haaren mit einem Farbstoffgehalt und für Haarfärbemittel üblichen Zusätzen, gekennzeichnet durch den Gehalt an einem Pikraminsäurederivat der allgemeinen Formel I

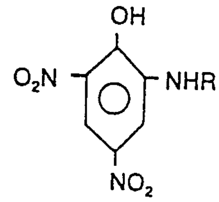

(I),

worin R einen Alkyl-, Hydroxyalkyl-, Dihydroxyalkyl-, Epoxyalkyl-, Alkoxyalkyl-, Aminoalkyl-, Alkylaminoalkyl- oder Dialkylaminoalkylrest bedeutet, wobei die Alkylfragmente jeweils einen unverzweigten und unsubstituierten Alkylrest mit insbesondere 2–3 Kohlenstoffatomen darstellen.

Die vorliegende Erfindung betrifft weiterhin neue Pikraminsäurederivate der allgemeinen Formel II

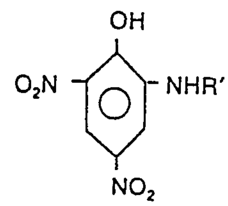

(II),

worin R′ Alkyl der Formel $C_nH_{(2n+1)}$ mit n = 2 bis 6 oder aber Hydroxyalkyl, Dihydroxyalkyl, Epoxyalkyl, Alkoxyalkyl, Aminoalkyl, Alkylaminoalkyl oder Dialkylaminoalkyl (wobei die Alkylfragmente jeweils einen unverzweigten und unsubstituierten Alkylrest mit insbesondere 2–3 Kohlenstoffatomen darstellen) bedeutet.

Die Pikraminsäurederivate der Formel I (welche die neuen Pikraminsäurederivate der Formel II einschließen) können nach einem neuen erfindungsgemäßen Verfahren hergestellt werden. Ihre Herstellung erfolgt durch Umsetzung der Pikraminsäure mit einem Alkylhalogenid R–X (X = Halogen), wobei R die vorstehend angegebenen Bedeutungen hat, in alkalischem Medium. Die Reaktion wird bei einer Temperatur von 100 bis 140 °C, vorzugsweise 120 °C, während einer Reaktionszeit von 1 bis 16 Stunden durchgeführt.

Beispiele für geeignete Alkylhalogenide sind Bromethan, 2-Chlorethanol, 2-Bromethanol, 1-Chlor-2,3-propandiol und 3-Brompropanol. Als Alkalisierungsmittel sind z.B. Alkalicarbonate wie Kaliumcarbonat oder Erdalkalicarbonate wie Calciumcarbonat geeignet, jedoch können auch andere basische Stoffe wie beispielsweise Triethylamin, Natriumhydroxid oder Natriumacetat eingesetzt werden. Als Lösungsmittel können die entsprechenden organischen Halogenide oder ihre wäßrigen Lösungen dienen. Bei der Verwendung von mit Wasser nicht mischbaren Halogeniden ist es von Vorteil, wenn als Lösungsmittel Glykolether, z.B. Glykoldiethylether, oder höhersiedende

Alkohole wie beispielsweise Methoxyethanol oder n-Butanol Verwendung finden.

Unter den genannten Reaktionsbedingungen erfolgt die Mono- und Dialkylierung der Pikraminsäure bevorzugt am Aminstickstoff. Die Trennung der Mono- und Dialkylierungsprodukte kann durch Kristallisation erfolgen. In manchen Fällen kann aber auch eine chromatographische Trennung des Reaktionsgemisches in die Einzelkomponenten von Vorteil sein.

Die anmeldungsgemäßen Verbindungen der Formel I stellen für die Färbung von Haaren hervorragend geeignete rotfärbende Nitrofarbstoffe dar. So eignet sich beispielsweise die neue erfindungsgemäße Verbindung 2-[(2′-Hydroxyethyl)-amino]-4,6-dinitrophenol besonders gut zur Erzeugung von modischen Rottönen. Ebensogut geeignet sind jedoch auch die ebenfalls neuen Verbindungen 2-[(3′-Hydroxypropyl)-amino]-4,6-dinitrophenol, 2-Ethylamino-4,6-dinitrophenol und 2-[(2′,3′-Dihydroxypropyl)-amino]-4,6-dinitrophenol.

Die vorstehend genannten neuen Verbindungen färben das Haar in einem reinen Rotton, ohne gelbe oder blaue Anteile. Diese Eigenschaft ist besonders für die Einstellung von Farbnuancen, bei denen gelbe bzw. blaue Farbanteile stören, von Vorteil. Überraschenderweise ist die ohnehin starke Farbintensität der erzielbaren Färbungen bei der Verwendung des 2-Ethylamino-4,6-dinitrophenols gegenüber dem 2-[(2′-Hydroxyethyl)amino]-4,6-dinitrophenol nochmals deutlich stärker.

Die erfindungsgemäßen Mittel zur Färbung von Haaren betreffen sowohl solche, die ohne Zugabe eines Oxidationsmittels angewendet werden, als auch solche, bei denen die Zugabe eines Oxidationsmittels erforderlich ist.

Bei den ersteren Haarfärbemitteln ohne Oxidationsmittel-Zugabe handelt es sich um diejenigen, die neben den Farbstoffen der angegebenen Formel I noch andere direkt auf das Haar aufziehende Farbstoffe enthalten können. Von diesen für die Haarfärbung bekannten Farbstoffen seien beispielsweise die folgenden erwähnt: Aromatische Nitrofarbstoffe, wie zum Beispiel 2-Amino-4-nitrophenol, 1-(2′-Hydroxyethylamino)-2-amino-4-nitrobenzol, 2-Nitro-4-(2′-hydroxyethylamino)-anilin, 1-Methylamino-2-nitro-4-di-(2′-hydroxyethyl)-aminobenzol und 2,5-Di-[(2-hydroxyethyl)-amino]-nitrobenzol; Triphenylmethanfarbstoffe, wie zum Beispiel Basic Violet 1 (C.I. 42 535); Azofarbstoffe, wie zum Beispiel Acid Brown 4 (C.I. 14 805); Anthrachinonfarbstoffe, wie zum Beispiel Disperse Violet 4 (C.I. 61 105), 1,4,5,8-Tetraaminoanthrachinon und 1,4-Diaminoanthrachinon, wobei die Farbstoffe dieser Klassen je nach Art ihrer Substituenten sauren, nichtionogenen oder basischen Charakter haben können. Weitere geeignete direkt auf das Haar aufziehende Farbstoffe sind beispielsweise in dem Buch von J.C. Johnson, «Hair Dyes» Noyes Data Corp. Park-Ridge (USA) (1973) beschrieben.

Die Zubereitungsform der hier beschriebenen Haarfärbemittel auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der angegebenen Formel I sollen in diesen Färbemitteln ohne Oxidationsmittel-Zugabe in einer Konzentration von 0,01 bis 2,0 Gew.%, vorzugsweise von 0,01 bis 1,0 Gew.%, enthalten sein. Der Gesamtgehalt an Farbstoffen liegt in den Grenzen von 0,01 bis 3,0 Gew.%.

Der pH-Wert dieser Färbemittel liegt im Bereich von 3 bis 10,5, insbesondere bei pH 7,5 bis 9,5, wobei die Einstellung des gewünschten alkalischen pH-Wertes hauptsächlich mit Ammoniak erfolgt, jedoch auch mit organischen Aminen wie beispielsweise Monoethanolamin oder Triethanolamin vorgenommen werden kann.

Ihre Anwendung erfolgt in üblicher Weise durch Aufbringen einer für die Haarfärbung ausreichenden Menge des Mittels auf die Haare, mit denen es eine Zeitlang, 5 bis 30 Minuten, in Berührung bleibt. Anschließend wird mit Wasser, gegebenenfalls noch mit einer wäßrigen Lösung einer schwachen organischen Säure, gespült und getrocknet. Als schwache organische Lösung können beispielsweise Essigsäure, Zitronensäure, Weinsäure und ähnliche Verwendung finden.

Die vorstehend beschriebenen Haarfärbemittel ohne Oxidationsmittel-Zugabe können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternisierungsprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von 1 bis 5 Gew.% enthalten. Die pH-Werte der Mittel liegen im Bereich von 6,0 bis 9,0.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich können die vorstehend beschriebenen Haarfärbemittel ohne Oxidationsmittel-Zugabe gegebenenfalls weitere gebräuchliche kosmetische Zusätze wie zum Beispiel Pflegestoffe, Netzmittel, Verdicker, Weichmacher und Parfümöle sowie auch andere, nachstehend für Oxidationshaarfärbemittel aufgeführte übliche Zusätze enthalten.

Zum Gegenstand der vorliegenden Erfindung gehören auch, wie eingangs erwähnt, diejenigen Haarfärbemittel, bei denen die Zugabe eines Oxidationsmittels erforderlich ist. Sie enthalten außer den Farbstoffen gemäß der angegebenen Formel I und gegebenenfalls bekannten direkt auf das Haar aufziehenden Farbstoffen noch zusätzlich bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole wie beispielsweise p-Toluylendiamin, p-Phenylendiamin, p-Aminophenol und ähnliche Verbindungen, welche zum Zwecke der Nuancierung der Färbungen mit sogenannten Modifiern, wie zum Beispiel m-Phenylendiamin, Resorcin, m-Aminophenol und anderen kombiniert werden.

Derartige zur Haarfärbung bekannte und übliche Oxidationsfarbstoffe sind unter anderem in dem Buch von E. Sagarin, «Cosmetics», Science and Technology (1957), Interscience Publishers Inc., New York Seiten 503 ff. sowie in dem Buch von H. Janistyn, «Handbuch der Kosmetika und Riechstoffe» (1973) Seiten 338 ff., beschrieben.

Mit Mischungen aus diesen Oxidationsfarbstoffen und den Farbstoffen gemäß der angegebenen Formel I lassen sich neben reinen Modetönen auch modische Blond- und Brauntöne erhalten.

Die Farbstoffe gemäß der Formel I sind in diesen Färbemitteln mit Oxidationsmittel-Zugabe in einer Konzentration von 0,01 bis 4,0 Gew.%, vorzugsweise 0,02 bis 2,0 Gew.%, enthalten. Der Gesamtgehalt an Farbstoffen in diesen Färbemitteln beträgt 0,1 bis 5,0 Gew.%.

Oxidationshaarfärbemittel sind im allgemeinen alkalisch eingestellt, vorzugsweise auf pH-Werte von etwa 8,0 bis 11,5, wobei die Einstellung insbesondere mit Ammoniak erfolgt. Es können dazu aber auch andere organische Amine, zum Beispiel Monoethanolamin oder Triethanolamin, Verwendung finden. Als Oxidationsmittel zur Entwicklung der Haarfärbungen kommen hauptsächlich Hydrogenperoxyd und dessen Additionsverbindungen in Betracht. Die Zubereitungsform dieser Haarfärbemittel kann die gleiche wie bei Haarfärbemitteln ohne Oxidationsmittelzugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate wie Carboxymethylcellulose, Alginate, Vaseline, Paraffinöl und Fettsäuren sowie außerdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von 0,5 bis 30 Gew.%, während die Verdicker in einer Menge von 0,1 bis 25 Gew.% in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt.

Nach einer für die Haarfärbung ausreichenden Einwirkungszeit, welche üblicherweise etwa 10 bis 45 Minuten beträgt, wird mit Wasser, gegebenenfalls anschließend mit der wäßrigen Lösung einer schwachen organischen Säure, wie zum Beispiel Zitronensäure oder Weinsäure, gespült und getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die nachstehenden Beispiele sollen den Anmeldungsgegenstand erläutern.

HERSTELLUNGSBEISPIELE

Beispiel 1

Herstellung von 2-Ethylamino-4,6-dinitrophenol (III)

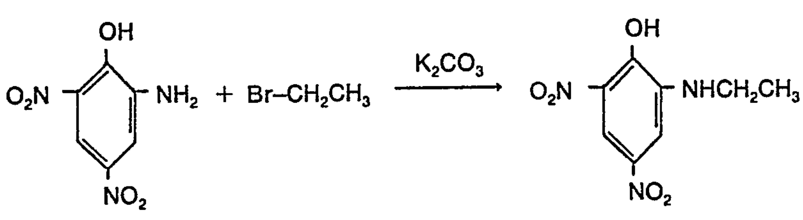


(III)

2,0 g (10 mMol) Pikraminsäure, 4,36 g (40 mMol) Bromethan und 2,76 g (20 mMol) Kaliumcarbonat werden mit 50 ml Methoxyethanol in einem Autoklaven während 7 Stunden auf 120 °C erwärmt.

Das Reaktionsgemisch wird dann mit 450 ml Wasser und 20 ml Natronlauge (2N) versetzt und mit Diethylether extrahiert. Danach wird die Wasserphase mit konzentrierter Salzsäure angesäuert und nochmals mit Ether extrahiert. Diese Etherphase wird mit Wasser gewaschen und über Magnesiumsulfat getrocknet. Man erhält daraus 1,45 g eines öligen Reaktionsproduktes. Nach der Kristallisation aus Ethanol erhält man 0,60 g (27%) des gewünschten Reaktionsproduktes (III) vom Schmelzpunkt 127–129 °C.

| CHN-Analyse | $C_8H_9N_3O_5$ | | |
|---|---|---|---|
| berechnet: | 42,30% C | 3,99% H | 18,50% N |
| gefunden: | 42,69% C | 4,09% H | 18,30% N |

Beispiel 2

Herstellung von 2-[(2'-Hydroxyethyl)-amino]-4,6-dinitrophenol (IV)

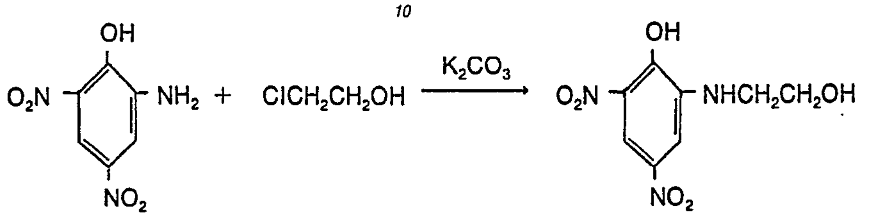


(IV)

4,0 g (20 mMol) Pikraminsäure, 10 g (72 mMol) Kaliumcarbonat und 100 ml 2-Chlorethanol werden für eine Stunde auf 120 °C erwärmt. Danach wird der Überschuß an Chlorethanol bei vermindertem Druck abdestilliert. Der Rückstand wird mit Salzsäure (2N) angesäuert und unter weiterem Erwärmen vollständig aufgelöst. Beim Abkühlen kristallisiert das gewünschte Produkt in orangeroten Plättchen aus. Die Ausbeute beträgt 2,8 g. Es kann zur weiteren Reinigung aus Ethanol kristallisiert werden. Das 2-[(2'-Hydroxyethyl)-amino]4,6-dinitrophenol (IV) weist einen Schmelzpunkt von 141–142 °C auf.

| CHN-Analyse | $C_8H_9N_3O_6$ | | |
|---|---|---|---|
| berechnet: | 39,51% C | 3,73% C | 17,28% N |
| gefunden: | 39,69% C | 3,86% H | 17,05% N |

Beispiel 3

Herstellung von 2-[(3'-Hydroxypropyl)-amino]-4,6-dinitrophenol (V)

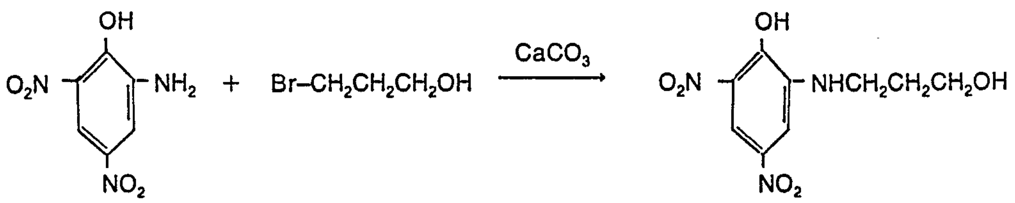


(V)

0,20 g (1 mMol) Pikraminsäure, 0,40 g (4 mMol) Calciumcarbonat, 0,65 g (5 mMol) 3-Brompropanol und 10 ml Wasser werden unter Rückfluß gekocht. Nach 16 Stunden wird abgekühlt und mit Salzsäure angesäuert. Die Wasserphase wird mit Ether extrahiert, die Etherphase wird mit Wasser gewaschen und über Magnesiumsulfat getrocknet. Man erhält daraus 0,2 g Produktgemisch, das an Kieselgel chromatographiert wird. Dadurch erhält man dann 20 mg eines reinen Produktes, das dem Haar die gleiche reine Rotfärbung verleiht wie das 2-[(2'-Hydroxyethyl)-amino]-4,6-dinitrophenol.

Beispiel 4

Herstellung von 2-[(2',3'-Dihydroxypropyl)amino]-4,6-dinitrophenol (VI)

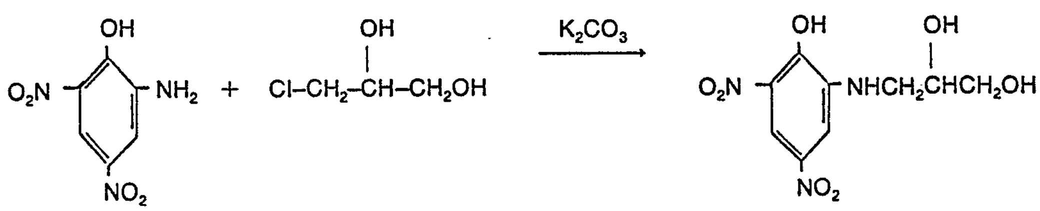


(VI)

20 g (0,1 Mol) Pikraminsäure, 50 g (0,36 Mol) Kaliumcarbonat und 200 ml 1-Chlor-2,3-propandiol werden zusammen eine Stunde lang auf 80 °C erwärmt. Nach dem Abkühlen wird der Ansatz mit 1,2 l Wasser verdünnt. Zur Entfernung von Nebenprodukten wird der Ansatz mit 100 ml 2n-Natronlauge versetzt und mehrmals mit Diethylether extrahiert. Die wäßrige Phase wird dann mit Salzsäure (d = 1,18) angesäuert. Das gewünschte Produkt kristalliert beim Abkühlen auf 0 °C aus. Es wird abgesaugt, mit Wasser gewaschen und über Calciumchlorid getrocknet. Ausbeute: 10,0 g (37% der Theorie).

Zur weiteren Reinigung wird das 2-[(2',3'-Dihydroxypropyl)amino]-4,6-dinitrophenol aus Wasser umkristallisiert. Es hat einen Schmelzpunkt von 155–156 °C.

| CHN-Analyse | $C_9H_{11}N_3O_7$ | | |
|---|---|---|---|
| berechnet: | 39,57% C | 4,06% H | 15,38% N |
| gefunden: | 39,30% C | 4,05% H | 15,10% N |

BEISPIELE FÜR HAARFÄRBEMITTEL

Beispiel 5

Färbelösung

| | |
|---|---|
| 0,3 g | Farbstoff nach Beispiel 1 |
| 2,0 g | Laurylalkoholdiglykolethersulfat-Natriumsalz (28%-ige wäßrige Lösung) |
| 2,0 g | Ammoniak, 25%-ige wäßrige Lösung |
| 95,7 g | Wasser |
| 100,0 g | |

Gebleichtes menschliches Naturhaar wird 20 Minuten lang bei Raumtemperatur mit der Lösung nach Beispiel 4 behandelt, danach wird mit Wasser ausgespült und getrocknet. Das Haar ist dunkelrot gefärbt.

Beispiel 6

Farbfestiger

| | |
|---|---|
| 0,1 g | Farbstoff nach Beispiel 2 |
| 2,0 g | Polyvinylpyrrolidon |
| 0,1 g | Glyzerin |
| 40,0 g | Isopropanol |
| 57,8 g | Wasser |
| 100,0 g | |

Weiße menschliche Haare werden mit der Farbfestiger-Lösung zur Frisur eingelegt und getrocknet. Das Haar ist rot gefärbt und gefestigt.

Beispiel 7

Oxidationshaarfärbemittel in Cremeform

| | |
|---|---|
| 0,40 g | 2,5-Diaminotoluolsulfat |
| 0,26 g | Resorcin |
| 0,40 g | 4-Aminophenol |
| 0,13 g | 2,4-Diaminoanisol |
| 0,50 g | Farbstoff nach Beispiel 2 |
| 0,30 g | Natriumsulfit, wasserfrei |
| 0,30 g | Ethylendiamintetraessigsäure-dinatriumsalz |
| 15,00 g | Cetylalkohol |
| 3,50 g | Laurylalkohol-diglykolethersulfat-Natriumsalz (28%-ige wäßrige Lösung) |
| 6,00 g | Ammoniak, 25%-ig |
| 73,21 g | Wasser |
| 100,00 g | |

50 ml des vorstehenden Haarfärbemittels werden kurz vor der Anwendung mit 50 ml Hydrogenperoxidlösung (6%-ig) gemischt. Das Gemisch wird anschließend auf graue menschliche Haare aufgetragen und 30 Minuten bei einer Temperatur von 40 °C einwirken gelassen. Nach dem Spülen des Haares mit Wasser und anschließendem Trocknen hat dieses einen Palisanderton angenommen.

**Patentansprüche**

1. Mittel zur Färbung von Haaren mit einem Farbstoffgehalt und für Haarfärbemittel üblichen Zusätzen, gekennzeichnet durch den Gehalt an einem Pikraminsäurederivat der allgemeinen Formel I

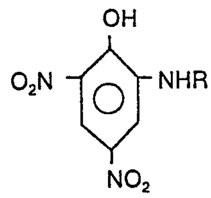

(I),

worin R einen Alkyl-, Hydoxyalkyl-, Dihydroxyalkyl-, Epoxyalkyl-, Alkoxyalkyl-, Aminoalkyl-, Alkylaminoalkyl- oder Dialkylaminoalkylrest bedeutet, wobei die Alkylfragmente jeweils einen unverzweigten und unsubstituierten Alkylrest mit insbesondere 2–3 Kohlenstoffatomen darstellen.

2. Mittel nach Anspruch 1, dadurch gekennzeichnet, daß R die Bedeutung Ethyl, 2′-Hydroxyethyl, 3′-Hydroxypropyl oder 2′,3′-Dihydroxypropyl hat.

3. Mittel nach den Ansprüche 1 und 2, dadurch gekennzeichnet, daß das Pikraminsäurederivat der Formel I in einer Menge von 0,01 bis 4,0 Gew.% enthalten ist.

4. Mittel zur oxidativen Färbung von Haaren nach den Ansprüchen 1 bis 3, zusätzlich enthaltend mindestens einen der bekannten Oxidationshaarfarbstoffe.

5. Mittel nach Anspruch 4, dadurch gekennzeichnet, daß es einen pH-Wert von 8,0 bis 11,5 aufweist.

6. Mittel in Form eines Haarfärbemittels mit zusätzlicher Haarfestigung nach den Ansprüchen 1 bis 2, zusätzlich enthaltend in wäßrig-alkoholischer Lösung bekannte, direkt auf das Haar aufziehende Farbstoffe und mindestens ein kosmetisches Polymerisat.

7. Pikraminsäurederivate der allgemeinen Formel II

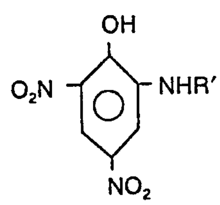

(II),

worin R′ Alkyl der Formel $C_nH_{(2n+1)}$ mit $n = 2$ bis 6 oder aber Hydroxyalkyl, Dihydroxyalkyl, Epoxyalkyl, Alkoxyalkyl, Aminoalkyl, Alkylaminoalkyl oder Dialkylaminoalkyl bedeutet, (wobei die Alkylfragmente jeweils einen unverzweigten und unsubstituierten Alkylrest mit insbesondere 2–3 Kohlenstoffatomen darstellen) bedeutet.

8. 2-Ethylamino-4,6-dinitrophenol nach Anspruch 7.

9. 2-[(2′-Hydroxyethyl)-amino]-4,6-dinitrophenol nach Anspruch 7.

10. 2-[(2′,3′-Dihyxdroxypropyl)-amino]-4,6-dinitrophenol nach Anspruch 7.

11. 2-[(3′-Hydroxypropyl)-amino]-4,6-dinitrophenol nach Anspruch 7.

**Claims**

1. Composition for colouring hair containing a dye and conventional additives for hair colouring compositions, characterised in that it contains a picramic acid derivative of the general formula I

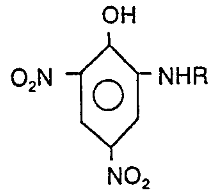

(I),

in which R means an alkyl-, hydroxyalkyl-, dihydroxyalkyl-, epoxyalkyl-, alkoxyalkyl-, aminoalkyl-, alkylaminoalkyl- or dialkylaminoalkyl residue, wherein each alkyl fragment represents an unbranched and unsubstituted alkyl residue, with in particular 2–3 carbon atoms.

2. Composition according to Claim 1, characterised in that R means ethyl, 2′-hydroxyethyl, 3′-hydroxypropyl or 2′,3′-dihydroxypropyl.

3. Composition according to the Claims 1 and 2, characterised in that the picramic acid derivative of formula I is present in a quantity of 0.01 to 4.0 weight %.

4. Composition for the oxidative colouring of hair according to Claims 1 to 3, also containing at least one of the known oxidation hair dyes.

5. Composition according to Claim 4, characterised in that it has a pH-value of 8.0 to 11.5.

6. Composition in the form of a hair colouring composition with additional hair strengthener according to Claims 1 to 2, also containing known direct absorption dyes for the hair and at least one cosmetic polymer, contained in an aqueous-alcoholic solution.

7. Picramic acid derivatives of the general formula II

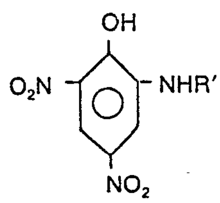

(II),

in which R′ means alkyl of the formula $C_nH_{(2n+1)}$ where n = 2 to 6 or alternatively means hydroxyalkyl, dihydroxyalkyl, epoxyalkyl, alkoxyalkyl, aminoalkyl, alkylaminoalkyl or dialkylaminoalkyl (wherein each alkyl fragment represents an unbranched and unsubstituted alkyl residue with in particular 2–3 carbon atoms).

8. 2-ethylamino-4,6-dinitrophenol according to Claim 7.

9. 2-[(2′-hydroxyethyl)-amino]-4,6-dinitrophenol according to Claim 7.

10. 2-[(2′,3′-dihydroxypropyl)-amino]-4,6-dinitrophenol according to Claim 7.

11. 2-[(3′-hydroxypropyl)-amino]-4,6-dinitrophenol according to Claim 7.

## Revendications

1. Composition de coloration pour cheveux, comprenant une teneur en matières colorantes et des adjuvants habituels pour les compositions de coloration des cheveux, caractérisé par une teneur en un dérivé d'acide picramique de formule générale I:

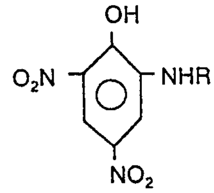

(I),

où R désigne un radical alkyle, hydroxyalkyle, dihydroxyalkyle, épоxyalkyle, alkoxyalkyle, aminoalkyle, alkylaminoalkyle ou dialkylaminoalkyle, et les fragments alkyles chaque designent un reste alkyle non ramifié et non substitué, particulièrement avec 2 à 3 atomes de carbon.

2. Composition selon la revendication 1, caractérisé en ce que R désigne éthyle, 2′-hydroxyéthyle, 3′-hydroxypropyle ou 2′,3′-dihydroxypropyle.

3. Composition selon les revendications 1 et 2, caractérisé en ce que le dérivé d'acide picramique de formule (I) est contenu selon une quantité comprise entre 0,01 et 4,0% en poids.

4. Composition de coloration oxydante de cheveux selon les revendications 1 à 3, contenant en plus au moins l'une des matières de coloration de cheveux par oxydation connues.

5. Composition selon la revendication 4, caractérisé en ce qu'il présente une valeur de pH de 8,0 à 11,5.

6. Composition se présentant sous la forme d'un agent de coloration des cheveux avec fixation additionnelle des cheveux selon les revendications 1 et 2, contenant en plus une matière colorante connue en solution aqueuse-alcoolique, absorbée directement par les cheveux, et au moins un produit de polymérisation cosmétique.

7. Derivé d'acide picramique de formule générale (II)

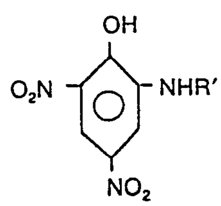

(II),

où R′ désigne un alkyle de formule $C_nH_{(2n+1)}$ avec n = 2 à 6, un hydroxyalkyle, dihydroxyalkyle, épоxyalkyle, alkoxyalkyle, aminoalkyle, alkylaminoalkyle ou dialkylaminoalkyle, et les fragments alkyles chaque désignent un reste alkyle non ramifié et non substitué, particulièrement avec 2 à 3 atomes de carbon.

8. 2-Ethylamino-4,6-dinitrophénol, selon revendication 7.

9. 2-[(2′-hydroxyéthyle)-amino]-4,6-dinitrophénol, selon revendication 7.

10. 2-[(2′,3′-dihydroxypropyle)-amino]-4,6-dinitrophénol, selon revendication 7.

11. 2-[(3′-hydroxypropyle)-amino]-4,6-dinitrophénol, selon revendication 7.